# EUROPEAN PATENT APPLICATION

(11) **EP 1 669 357 A1**
(43) Date of publication of application: **14.06.2006**
(21) Application number: 03810030.1
(22) Date of filing: 05.11.2003
(51) Int. Cl.: C07D 491/147, C07D 471/14, A61K 31/519, A61P 31/00

(54) **SUBSTANCE WHICH EXHIBITS ANTIVIRAL AND ANTIBACTERIAL ACTIVITY AND IS BASED ON DERIVATIVES OF 2,8-DITHIOXO-1H-PYRANO 2,3D&cedi l;6,5-D DIPYRIMIDYNE AND 10-AZA-ANALOGUE THEREOF**

(30) Priority: 12.09.2003 RU 2003128343
(71) Applicant: Tets, Viktor Veniaminovich, St. Petersburg, 196066 (RU); Krasnov, Konstantin Andreevich, St. Petersburg 199155 (RU); Ashkinazi, Rimma Iliinichna, St. Petersburgm, 191025 (RU)
(72) Inventor: TETS, Viktor Veniaminovich, St.Petersburg, 196066 (RU); KRASNOV, Konstantin Andreevich, St.Petersburg, 199155 (RU); ASHKINAZI, Rimma Iliinichna, St.Petersburg, 191025 (RU)
(74) Representative: Schneider, Henry
(86) International application number: PCT/RU2003/000496
(87) International publication number: WO 2005/026171

(57) **Abstract**

The inventive substance which exibits antiviral and antibacterial activity is based on derivatives of 2,8-dithioxo-1H-pyrano[2,3-d; 6,5-d']dipyrimidine and 10-aza-analogue, differs in that comprises the derivative of said group of general formula A1 * M: 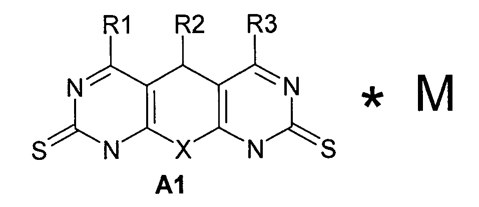 where: X is selected from a group of O, NH, N-Alkyl;
R1 is selected from the group of H, OH, Cl, O-Alkyl, NH₂, NH-Alkyl, NH-Ar, N(Alkyl)₂, SH, S-Alkyl, S-Ar, S-Hetaryl;
R2 is selected from the group of C6H5, Aryl;
R3 is selected from the group of H, Cl, O-Alkyl, NH2, NH-Alkyl, NH-Ar, S-Hetaryl;
M-is absent or is selected from a group: cation Na, K, Li, ammonium or any other pharmacologically acceptable cation or complex of pharmacologically acceptable cation (see above) with anion of one of derivatives of A1 (variants R1-R3 are given above).

## Description

### Technical field

The invention relates to organic chemistry and medicine and, more specifically to synthetic pyrimidine derivatives i.e. 2,8-dithioxo-1H-pyrano[2,3-d; 6,5-d']dipyrimidine and their 10-aza-analogue thereof and to the complexes and salts thereof which exhibit antiviral and antibacterial activities. Said invention can be used for medicine and veterinary science for treatment viral diseases, bacteria-induced diseases and for cosmetology in the form of an additive for preventing and treating infections.

### Background art

It is well known that many of pyrimidine derivatives are biologically-active substances and take place in many processes [1- Dorson R. et al. Biochemist' desk book. Moscow. Mir, 1991, 544]. Synthetic derivatives of pyrimidine are widely used in medicine, in particular substituted barbituric acids, uraciles e.t.c [2- Mashkovsky M.D.drug compositions, 14-th edition., Moscow., "New Wave", 2000]. Data on a biological activity of various derivatives of 5-ilidenbarbituric acids was summarized in the review [3- Sans R.G., Chosas M.G. // Pharmazie, 1988, Bd 43, N 12, S. 827-829],], in which anticonvulsive, antimicrobial, spasmolytic, antipyretic, and antitumoral effects of these substances were noted.

Data was also obtained on biological activity of some 5-arylidenbarbituric acids [Singh A. 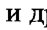 p. // Pharmacol. Res., 1989, Vol. 21, N 1, P. 59-64 (Chemical Abstracts, Vol. 11, 49906z); Patent in Japanese No 05213755, publ. 24.08.1993 (Chemical Abstracts,1993, Vol. 119, 262520r); Kumar A., et al. // Indian J., Chem. Sect. 1988, Vol. 27, N 5, P. 443-447], 5-aminomethylenebarbituric acids [9-.Kreutzberger A. Et al. // Arch. Pharm., 1983, Bd 316, H. 1, S. 6-9], 5-arylcarbamoylbarbituric acids that are the products of reactions of barbituric acids with isocyanates or isothiocyanates [10, 11- Minatelli J.A. et al. Claimed in Germany Nº 3446371, publ. 27.06.1985; Brewer A.D. Patent in USA Nº 4920126, publ. 24.04.1990].

High activity was observed at annelated derivatives of pyrimidine, for example pyrasole[3,4-d]pyrimidines [12-Naka T. Et al. claim European patent organization Nº. 237289 (1987)], 5-deazaflavins [13, 14-.Kimachi T. 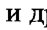 p. // J. Heterocycl. Chem., 1992, Vol. 29, N 4, P. 763-765;, 5-dialkylaminomethyluridines [19- Motawia M.S. et al. // Monatsh. Chem., 1993, Bd 124, H. 1, S. 55-64] and pyrimido[4,5-c]pyridazines [20- Billings B.K. et al. // J. Heterocycl. Chem., 1975, Vol. 12, N 6, P. 1221-1224]. The compounds listed possess pesticide, antitumoral, antimicrobial, immunosuppressive, nootropic, antihypertensive, and anti allergic activity.

At the same time, many groups of pyrimidine derivatives still to be practically unstudied because of their synthesis' difficulty and absence of objective criteria allowing to foresee levels of derivatives' possible activity, toxicity and side effects. Thus in spite of theoretical and practical difficulties synthesis of new pyrimidine' derivatives and study of their biological activity still to be actual direction of new effective ways of human main diseases' treatment. According to it synthesis of derivatives of poor-studied group of pyrano[2,3-d:6,5-d']dipyrimidine is extremely interesting.

There are a few examples only of producing pyranodipyrimidine system: when reacting of barbituric acids with 3-acylchromones [21, 22- Eiden F. et al. // Chemische Berichte, 1968, Bd 101, N 8, S. 2894-2898; Eiden F. et al..// Arch. Pharm., 1972, Bd 305, N 3, S. 187-193]. Data on their biological activity is absence.

Derivatives of 5H-pyrano[2,3-d:6,5-d']dipyrimidine possessing antibacterial, antiviral and immunomodulating effects [23- Achkinazi R.I. PCT/RU97/00371 from 19.11.1997, patent RU 2188201] are the most close to the claimed compound. Theses compounds were taken as prototype. At the same time, compounds from prototype group possess limited spectrum of antiviral and antibacterial activity: they are not active against influenza viruses and possess low activity against retroviruses.

### Summary of the invention

Objective of the invention is to develop a new more effective chemical compounds possessing antiviral, and antibacterial effects.

The proposed objective is achieved by synthesis of new compounds of general formula A1* M: 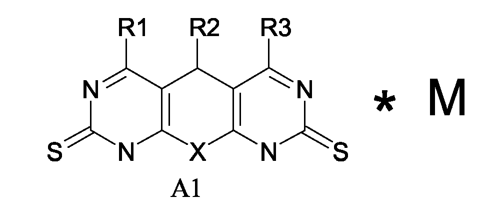 where: X is selected from the group of O, NH, N-Alkyl;
R1 is selected from the group of H, OH, Cl, O-Alkyl, NH₂, NH-Alkyl, NH-Ar, N(Alkyl)₂, SH, S-Alkyl, S-Ar, S-Hetaryl;
R2 is selected from the group of C₆H₅, Aryl;
R3 is selected from the group of H, Cl, O-Alkyl, NH₂, NH-Alkyl, NH-Ar, S-Hetaryl;
M-is absent or is selected from the group: cation Na, K, Li, ammonium or any other pharmacologically acceptable cation or complex of pharmacologically acceptable cation (see above) with anion of one of derivatives of A1 (variants R1-R3 are given above).

The best activity of claimed compounds A1*M (where M is absence) have shown the following derivatives:

| Number | X | R1 | R2 | R3 |
|---|---|---|---|---|
| la | O | OH | C₆H₅ | Cl |
| Ib | O | OH | C₆H₄-4-NO₂ | Cl |
| Iia | O | Cl | C₆H₄-4-NO₂ | Cl |
| III | O | OH | C₆H₄-4-NO₂ | NH₂ |
| IV | O | Cl | C₆H₄-4-NO₂ | NH₂ |
| V | O | NH₂ | C₆H₄-4-NO₂ | NH₂ |
| VI | NH | NH₂ | C₆H₄-4-NO₂ | NH₂ |
| VII | NH | OH | C₆H₄-4-Cl | NH₂ |
| VIII | O | OH | C₆H₄-4-NO₂ | NHCH₃ |
| IX | O | OH | C₆H₄-4-NO₂ | N(CH₃)₂ |
| X | O | OH | C₆H₄-4-NO₂ | 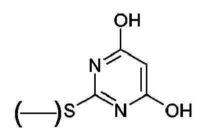 |
| XI | O | OH | C₆H₄-4-NO₂ | H |
| XII | O | OH | C₆H₄-4-NO₂ | OH |

The following complexes of A1*M were also included in the group with the highest activity level:
XIII - Complex salt that consists of: {Ib (1 mole), XII (1 mole), NH₃ (1 mole)}
XIV - Complex salt that consists of: {III (1 mole), XII (1 mole), NH₃ (1 mole)}
XV - Complex salt that consists of: {XI (1mole), XII (1 mole), NH₃ (1 mole)}

As it is seen from materials listed above, claimed derivatives of pyrano[2,3-d:6,5-d']dipyrimidine A1*M differs from prototype by other functional groups in pyrimidine fragments and can not be achieved by methods indicated in prototype. The applicant has not found sources that contained data about technical decision identical to the present invention that allows conclude that invention correspond to the criteria of "novelty" (N).

It is necessary to indicate that high biological activity of claimed compounds is not obvious from existing technical level because pyrano[2,3-d:6,5-d']dipyrimidines and 10-aza-analogues is complicated and poorly-investigated group for new members of which it is impossible to predict spectrum and activity level.

It is necessary to mention that the invention subsists not only on the most active compounds (Ia,Ib, IIa, II-XII) and their salt-complexes (XII-XV) but also on all derivatives of A1*M foreseen by invention' formula. Investigations held by us have shown that all obtained compounds synthesized by general method that is claimed by us, possess listed levels of activities. It makes possible to conclude that for synthesis and for biological properties of claimed compounds, the most significant role belongs not to the structure of R1, R2, R3 radicals but to their relevance to chemical groups listed in general formula.

No sources of information, that contained data of influence of claimed distinguishing features on achieved by their usage technical result were found by applicant. According to applicant, this fact testifies correspondence of given technical solution to criteria of "inventive step" (IS).

### Brief description of the drawings

The invention is elucidated in detailed description of examples of its realization without references to drawings.

### Preferred embodiment

The subject matter of the present invention is explained below by data about synthesis and analysis of claimed compound and its biological properties that were experimentally determined.
A) Data on synthesis and analysis of claimed compound contains description of two stages of synthesis' general method and description of 10 examples of realization of synthesis.
   Physico-chemical properties of synthesized compounds are presented in two tables. Table 1 contains spectrums of Paramagnetic Resonance (PMR) of synthesized compounds; table 2 - decomposition points, and data of element analysis.
B) Experimental determination of biological properties of claimed compounds contains description of six examples of their antiviral and antibacterial activity that was experimentally determined.

### A. Synthesis and analysis data of claimed compound.

The general method of synthesis of claimed compounds (2 stages).

### Proposed method of synthesis consists of 2 main stages:

The first stage: Synthesis of 5-aryl derivatives 4-clorine-6-hydroxi-5-aryl-5,9-dihydro-1H-pyrano[2,3-d;6,5-d']dipyrimidine-2,8-dition (IIb) or 4,6-dichlorine-1H-pyrano[2,3-d; 6,5-d']dipirimidine-2,8-dition (IIa) from correspondent aromatic aldehydes and 2-thiobarbituric acid with subsequent processing by POCl₃ or another chlorinating and dehydrating agents. Intermediate compounds of this synthesis are (Ia, Ib , IIa and their analogues) at the same time are relevant, as possess high level of biological activity.

The second stage. Synthesis of relevant compounds (III-V, VIII-XII and their analogues, where X=0, R1=OH are substituted or non-substituted group, R2=is substituted or non-substituted benzoic ring, R3 - substituted or non-substituted amino group or alkoxy group, substituted or non-substituted mercapto group) from intermediate compounds received on the first stage of synthesis (I and II), by substitution of one or two atoms of chlorine in tricyclic 1H-pyrano[2,3-d; 6,5-d']dipirimidine-2,8-ditione system on NH₂-, alkylamino-, or dialkylamino group, alkoxy group, SH-, alkythio-, arylthio- or hetarylthio group by processing by specific nucleophilic reagents (amines, alcoholates or thiolates);

New relevant compounds (VI, VII and their analogues, where X=NH or N-Alkyl, R1=OH, substituted or non-substituted amino group, R2- substituted or non-substituted benzoic ring, R3 - substituted or non-substituted amino group) are synthesized from intermediate compounds received on the first stage of synthesis (Ia, b, and IIa) by substitution of one or two atoms of chlorine in tricyclic 1H-pyrano[2,3-d; 6,5-d']dipirimidine-2,8-ditione system on NH₂- or alkylamino group with simultaneous exchange of pyranic atom of oxygen (O10) on amino group;

Relevant compounds (XI and its analogues, where R2- substituted or non-substituted benzoic ring) are synthesized from intermediate compound received on the first stage of synthesis (Ia) by its reductive dehalogenation;

Relevant compounds (complex salts XIII-XV) are synthesized by dissolving of equimolar quantities of compounds II and XII or XI and XII or Ib and XII in the excess of ammonia with subsequent acidifying of solution.

Example 1. Version of synthesis of the first stage - synthesis of intermediate (and simultaneously relevant) product, in particular - 4-chlorine-6-hydroxi-5-aryl-5,9-dihydro-1H-pyrano[2,3-d;6,5-d']dipyrimidine-2,8-dition (Ia)

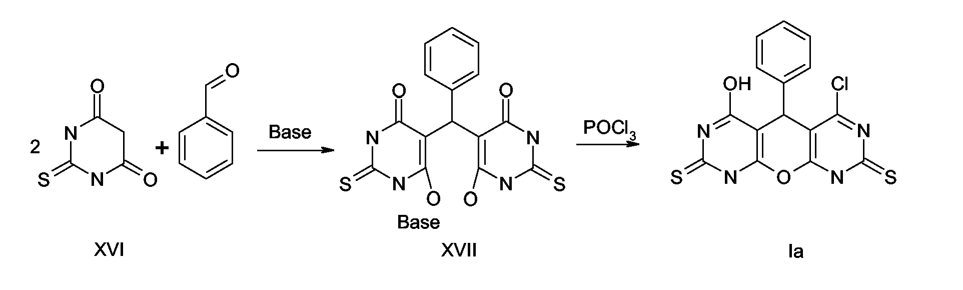

0,1 mole of 2-thiobarbituric acid (XVI) is dissolved in 50 ml of dimethylacetamide. The mixture of 0,05 mole of benzaldehyde * and 0,05 mole of base (triethylamine) in dimethylacetamide solution is added to the first solution and total solution is mixed. Several hours later, ether is added. The sediment is washed by ether and dried by tetraethylammonium salt (XII). 0,3 mole of POCl₃ and 100 ml of chloroform is added to the received salt XII (27g) and is boiled for 3 hours. After it the solvent is distilled, warm water is added to the residue; solid substance is separated, washed with water and dried. The yield of Ia is about 81 %.

*Note. 4-chlorine-6-hydroxi-5-(4-nitrophenyl)-5, 9-dihydro-1H-pyrano[2,3-d;6,5-d']dipyrimidine-2,8-dition (Ib) and 4-chlorine-6-hydroxi-5-aryl-5(4-chlorphenyl)-5,9-dihydro-1H-pyrano[2,3-d;6,5-d']dipyrimidine-2,8-dition (Ic) are received by the same technique (the yield is about 8% and 77%) using h-nitrobenzolealdehyde instead of p- chlorine benzaldehyde correspondingly. Using of another aromatic aldehydes results in correspondent 5-arylderivatives 4-chlorine-6-hydroxi-5,9-dihydro-1H-pyrano[2,3-d; 6,5-d']dipirimidine-2,8-dithione (see general formula, X=O, R1= OH, R2= Aryl, R3= Cl, M - no).

We do not specify these variants, listing only the most active representatives (Ia, Ib), listed in table 1.

Example 2. Version of synthesis of the first stage ― synthesis of intermediate and simultaneously relevant) product, in particular 4,6-dichlorine-5-(4-nitrophenyl)-5,9-dihydro-1H-pyrano[2,3-d; 6,5-d']dipyrimidine-2,8-dition (IIa).

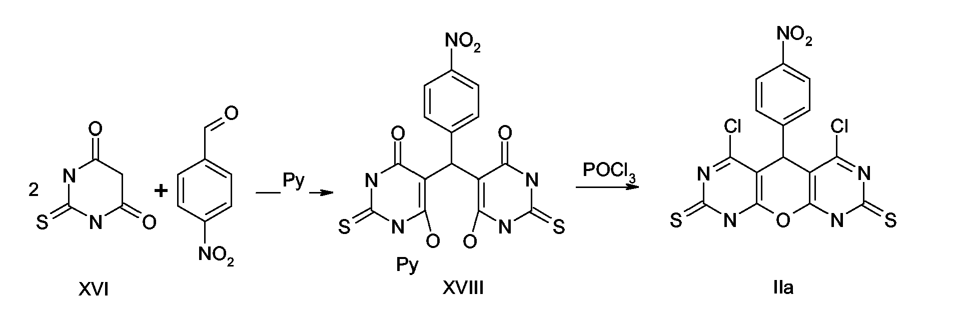

To 0,1 mole of 2-thiobarbituric acid (XII) is dissolved in 50 ml of pyridine. 0,05 mole of p- nitrobenzolealdehyde. The mixture is heated up to the full dissolving of reagents. Several hours later, ether is added. The sediment is washed by ether and pyridine acid and dried (XVIII) .

To the received salt XVIII (26,5 g), 0.5 mole of POCl₃* is added and heated with reflux condenser during 1 hour until total solution of the sediment. After it, excess of POCl₃ is distilled, the water is added, deposit precipitation is separated, washed by water and dried. To this compound, 0,5 mole of POCl₃ is added again and the procedure written above is repeated. After washing and drying, product IIa is received. The yield of product is 77%

*Note. 4,6-hydroxi-5,9-dihydro-1H-pyrano[2,3-d;6,5-d']dipyrimidine-2,8-dition XII and its analogues are received by the same technique, using trifluoroacetic acid anhydride instead of POC13 (see general formula, X=O, R1=R3+OH, R2=Aryl, M is absent)

Example 3. Is a version of synthesis of the second stage ― synthesis of relevant product, in particular - 4-amino-6-hydroxi-5-(4-nitrophenyl)-5,9-dihydro-1H-pyrano[2,3-d;6,5-d']dipyrimidine-2,8-dition (III).

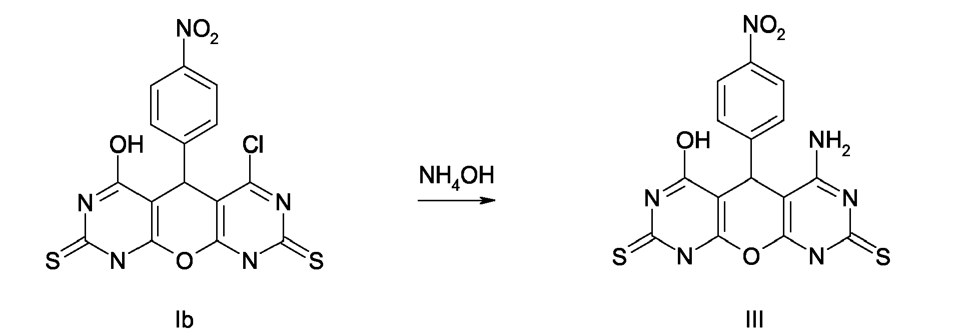

0,01 mole of compound Ib is mixed and diluted in 30 ml of 25% ammonia*. Not dissolved compound is separated and solution is stored at room temperature for 24 hours and then is diluted by water and acidified up to pH 5-6. Separated sediment is washed by water, spirit and dried. The yield of III is about 81%.

Note*. 4-methylamino-6-hydroxi-5-(4-nitrophenyl)-5,9-dihydro-1H-pyrano[2,3-d;6,5-d']dipyrimidine-2,8-dition VII is received by the same technique, using methylamine instead ammonia, the yield of product is 78% and 4-dimethylamine-6-hydroxi-5-(4-nitrophenyl)-5,9-dihydro-1H-pyrano[2,3-d; 6,5-d']dipyrimidine-2,8-dition (IX). The yield of product is 73%. The method described in example 3, is general and allows to receive correspondent 5-aryl-4-amino derivatives 6-hydroxi-5,9-dihydro-1H-pyrano[2,3-d; 6,5-d']dipirimidine-2,8-dition (see general formula, X=O, R1=OH, R2=substituted or non-substituted phenyl and other aryl, R3=NH2 or NHAlk and NHAlk2, M is absent) by exchanging chlorine atom in reaction with ammonia (or alkylamines) on amine group. We do not list all these variants, listing only most active representatives(III, VIII, IX), indicated in table 1.

Example 4. Is a version of synthesis of the second stage ― synthesis of relevant product, in particular 4-chlorine-6-amino-5-(4-nitrophenyl)-5,9-dihydro-1H-pyrano[2,3-d; 6,5-d']dipyrimidine-2,8-dition (IV).

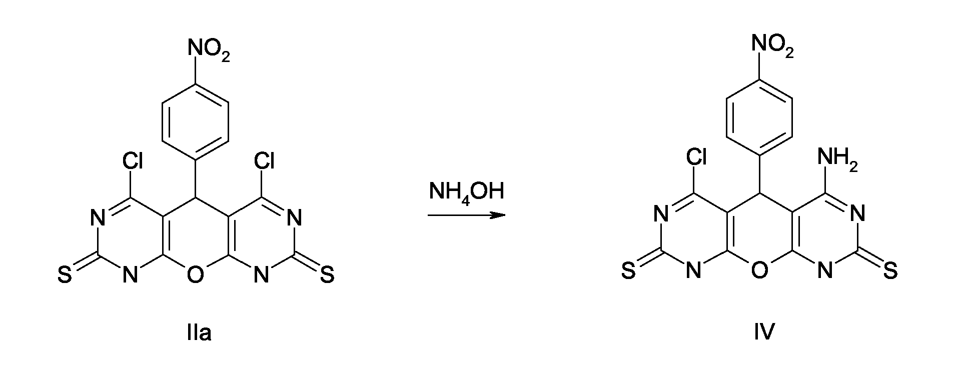

10 ml of ethanol, that contains 0,02 mole of NH₄OH is added to 0,01 mole of IIa compound and mixed. The mixture is incubated for 24 hours at room temperature, than diluted by water and acidified to pH 5-6. The deposit precipitation is separated washed by water and dried. The yield of product is 54%.

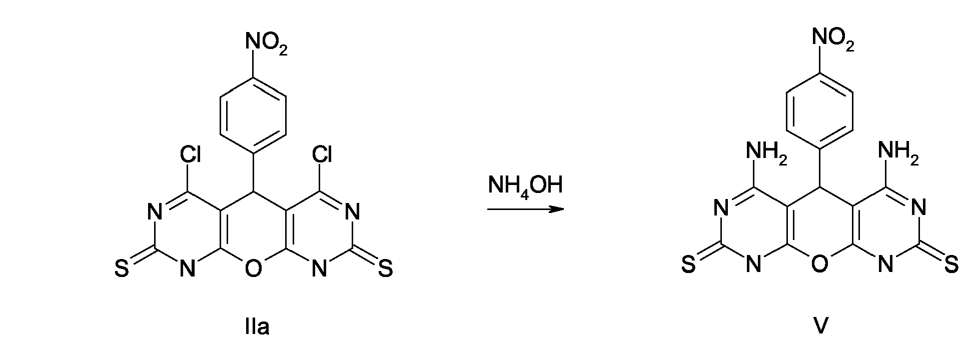

Example 5. is a version of synthesis of the second stage - synthesis of relevant product, in particular 4,6-diamino-5-(4-nitrophenyl)-5,9-dihydro-1H-pyrano[2,3-d; 6,5-d']dipyrimidine-2,8-dition (V)*.

0.01 mole of compound IIa is mixed is diluted in 30 ml of 25% ammonia. Not diluted compound is separated, solution is incubated at room temperature, diluted by water and acidified up to pH 5-6. The sediment is separated, washed by water, spirit and dried. The yield of product is 66%.

*Note. Method described in Example 5 is general and allows to substitute in 5-aryl derivatives of 4,6-dichlorine-5,9-dihydro-1H-pyrano[2,3-d 6,5-d']dipyrimidine-2,8-dition chlorine atoms in reaction with ammonia (or alkylamines) on amino groups and receive correspondent 5-aryl-4,6-diaminoderivatives 5,9-dihydro-1H-pyrano[2,3-d; 6,5-d']dipyrimidine-2,8-dition (see General Formula where X=O, R1,R3=NH₂ or NHAlk, or Nalk2, R2=nonsubstituted or substituted phenyl or another aryl, M is absent). We do not list all these variants, listing only most active representatives(III, VIII, IX), indicated in table 1.

Example 6. Is a version of synthesis of the second stage - synthesis of relevant product, in particular 4,5-diamino-10-(4-nitrophenyl)-9,10-dihydro-1H,8H-1,3,6,8,9-pentaazaantrecen-2,7-dition (VI).

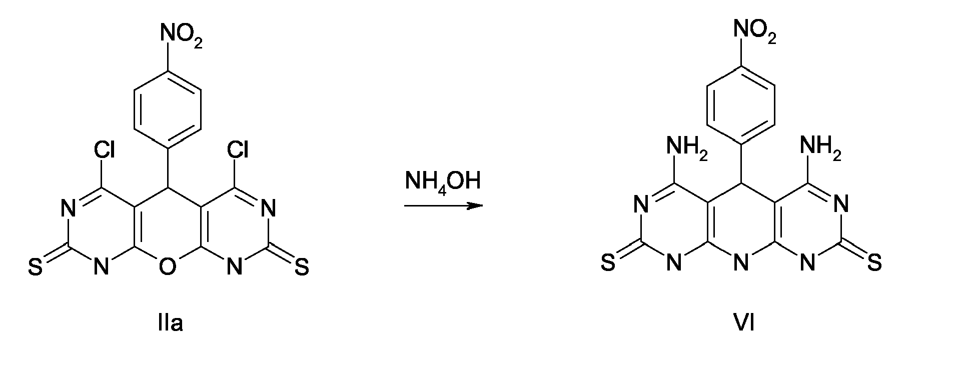

0,01 mole of compound IIa is diluted in 80 ml of 25% ammonia. Not diluted particles of the solution are filtered and heated during 24 hours with a reflux condenser. After it the solution is boiled until its volume become 40 ml, cooling, washed with ethanol, and dried. The yield is 31%.

Note. The same method is used for receiving 4-hydroxi-5-amino-10-(4-chlorphenyl)-9,10-dihydro-1H8H-1,3,6,8,9-pentaazaantracen-2,7-dition (VII) from compound Ic (see Example 1).

Example 7. is a version of synthesis of the second stage ― synthesis of relevant product, in particular 4-(4,6-dihydroxipirimidine-2sulfanil)-6-hydroxi-5-(4-nitrophenyl)-5,9-dihydro-1H-pyrano[2,3-d; 6,5-d']dipyrimidine-2,8-dition (X).

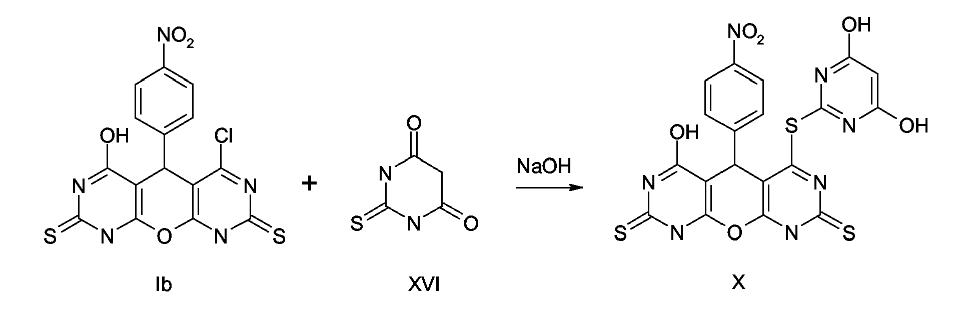

0,015 mole of 2-thiobarbituric acid (XVI) non stop mixing is added to 20 ml of water that contains 0,03 mole of NaOH. 20 ml of dimethyl sulfoxide is added to the received solution and after that 0.01 mole of compound Ib in dimethyl sulfoxide is added. Received solution is mixed for several hours at room temperature. The solution is diluted by water, acidified up to pH 5-6.. Deposited precipitation is filtered and washed with water, ethanol and dried. The yield is 42%.

Example 8. is a version of synthesis of the second stage ― synthesis of relevant product, in particular 6-hydroxi-5-(4-nitrophenyl)-5,9-dihydro-1H-pyrano[2,3-d; 6,5-d']dipyrimidine-2,8-dition (XI).

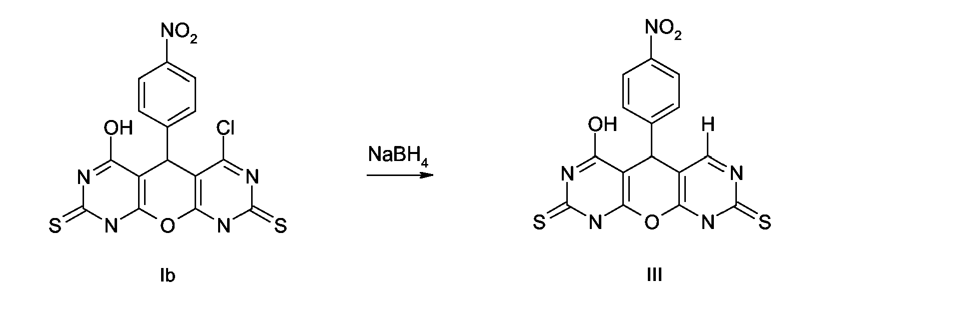

0.01 mole of compound Ib is diluted in 25 ml of concentrated acetic acid. 0,02 mole of NaBH₄ is fractionally added to this solution during 0.5 hours at temperature not more than 30°C. The solution is mixed for 4 hours and poured in water.. Deposited precipitation is filtered and washed with water, ethanol and recrystallizing from dimethylformamide. The yield is 36%.

Example 9. is a version of synthesis of the second stage - synthesis of relevant product, in particular complex salt XIII, that consists of compound Ib (1 mole), compound XII (1 mole) and NH₃ (1 mole).

0.01 mole of compound Ib* and 0.01 mole of compound XII is diluted non stop mixing without heating in 200 ml 0.5% of ammonia. Non diluted precipitate is filtered and processed by new portion of 50 ml 0.5% of ammonia. United, transparent solution is acidified by acetic acid and incubated for several hours at room temperature. Deposited precipitation is filtered and washed with water, ethanol and dried. The yield of salt XII is 80%.

*Note 1. Salts XIV and XV are received by the same method, using compounds II and XI correspondingly instead of lb.

Example 10. is a version of synthesis of the second stage - synthesis of relevant product, in particular complex salt XIV, that consists of compound III (1 mole), compound XII (1 mole) and NH₃ (1 mole).

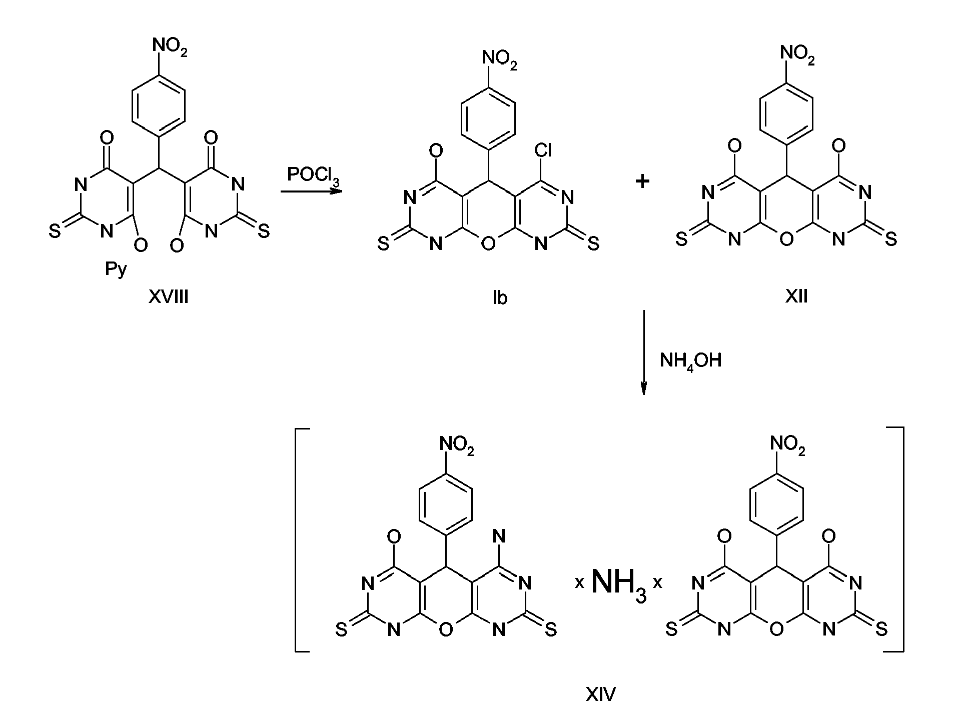

12 ml of POCl₃ is added to 0.01 mole of pyridine salt XVIII (see Example 2) and heated reflux condenser during 40-50 minutes until the main part of precipitate will not get into solution. The solution is decantined from the precipitate, 5 ml POCl₃ is distilled and poured into ice, deposit precipitation is distilled and washed by water. 40 ml of water and 10 ml of concentrated ammonia is added to the received product and mix for 4 hours without heating. Most part of the precipitate get diluted. The solution is filtered and acidified by acetic acid. Several hours later, sediment is filtered, washed by water and dried. The yield of product is 71 %.
Physical-chemical properties of synthesized compounds are presented tables 1 and 2: spectrums of Paramagnetic Resonance of synthesized compounds are presented in table 1; decomposition points, and data of element analysis are presented in table 2.

Examples 1-8 of practical synthesis, and physical-chemical characteristics of the synthesized compounds presented in tables 1 an 2 confirm the possibility of laboratory and industrial synthesis of claimed compound at all points of claims by the means that have been leant by modem pharmaceutical industry, and also their clear identification by generally used control methods.

### Physical-chemical characteristics of claimed compounds.

**Table 1. PMR spectra of solutions of the claimed compounds, in DMSO-d6 (δ, ppm, J, Hz)**

| Nº | C(5)H 1H, c* | Ar | NH (OH) es | NH₂ (NMe) Es |
|---|---|---|---|---|
| la | 5.02 | 6.79 (1H, t), 7.16 (2H, t), 7.33 (2H, d) J 8.0 | 11.05 (1H), 12.44 (1H), 13.55 (1H) | - |
| lb | 5.03 | 7.54 (2H, d) 8.02 (2H, d), J 8.4 | 11.18 (1H), 12.35 (1H), 13.40 (1H) | - |
| lc | 5.01 | 7.14 (2H, d) 7.45 (2H, d), J 8.4 | 11.11 (1H), 12.20 (1H), 13.35 (1H) | - |
| lia | 5.39 | 7.56 (2H, d) 8.04 (2H, d), J 8.3 | 12.91 (2H) | - |
| III | 4.94 | 7.50 (2H, d) 7.92 (2H, d), J 8.3 | 11.80 (2H), 12.84 (1H) | 11.96 (2H) |
| IV | 5.10 | 7.52 (2H, d) 8.00 (2H, d), J 8.0 | 12.70 (2H) | 12.25 (2H) |
| V | 4.99 | 7.48 (2H, d) 7.89 (2H, d), J 8.2 | 12.84 (2H) | 11.90 (4H) |
| VI | 4.54 | 7.52 (2H, d) 8.00 (2H, d), J 8.4 | 12.90 (3H) | 11.66 (4H) |
| VII | 4.66 | 7.16 (2H, d) 7.33 (2H, d), J 8.0 | 11.15 (1H), 12.91 (2H) | 12.34 (2H) |
| VIII | 4.85 | 7.52 (2H, d) 8.04 (2H, d), J 8.4 | 11.20 (1H), 12.45 (3H) | 3.59 (3H, s) |
| IX | 4.95+5. 19 | 7.65 (2H, d), 7.86 (4H, m), 8.07 (2H, d), J 8.0 | 11.10 (2H), 12.85 (3H) | - |
| X | 4.92 s | 5.28 (1 H, s), 7.56 (2H, d) 8.04 (2H, d), J 8.1 | 11.23 (1H), 12.35 (4H) | - |
| XI | 5.02 | 7.55 (2H, d) 7.97 (2H, d), 8.31 (1 H, s) | 11.14 (1H), 12.80 (2H) | - |
| XII | 4.75 | 7.65 (2H, d) 8.11 (2H, d), J 8.0 | 12.55 (2H), 13.9 (2H, es) | - |
| XIII | 4.80 es 5.25 es | 7.55 (4H, es) 8.10 (4H, es) | 9.11 (1H es), 11.05 (2H, es); 12.45 (2H, es) | 7.25 (4H es) |
| XIV | 4.95 es 5.13 es | 7.53 (4H, es) 8.12 (4H, es) | 10.00 - 13.50 (5H, es) | 7.24 (4H es) |
| XV | 4.95 es 5.13 es | 7.50 (4H, es) 8.07 (4H, es); 8.35 (1 H, es) | 9.70-13.40 (5H, es) | 7.25 (4H es) |

| | | | | |
|---|---|---|---|---|
| s - singlet, es - extended singlet, d - doublet, t - triplet, m multiplet | | | | |

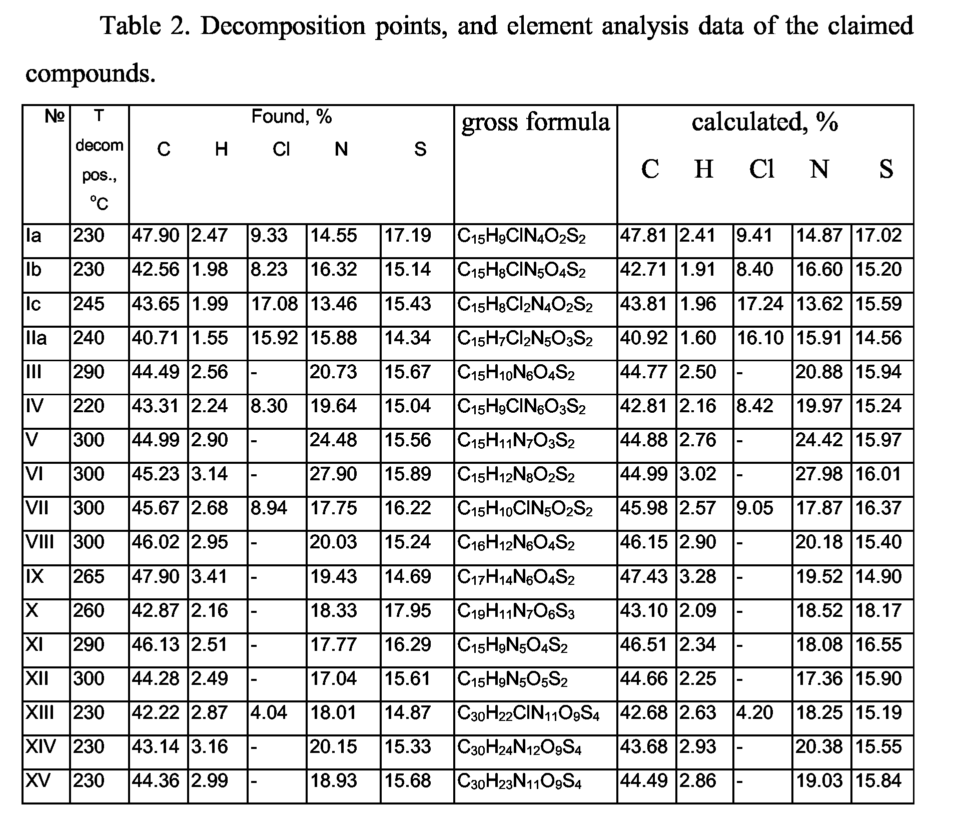

### B. Experimental determination of biological effects of claimed compounds

Example 11. Determination of effect of the claimed compounds on *Herpes virus.* An antiviral activity was determined with respect to Herpes virus of the type I (SHV-I /Leningrad/248/88) by means of generally accepted method [ 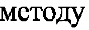 [24 - Gentry G.A. 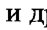 . J. of Clinical Microbiology, 1985, 22, 2, P.199-204]. Viruses were grown on a continuous culture of Vero cells that have been received from the Bank of Cell Cultures of the Institute of Cytology of the Russian Academy of Science.

**Table 3. Effect of the claimed compounds on the Simple Herpes Virus.**

| NN | Compound | Number of cells | | |
|---|---|---|---|---|
| | | 100* | 50* | 10* |
| 1 | Acyclovir | -** | - | 9600 ***(80%)**** |
| 2 | DMCO | 10000 | 10000 | 10000 |
| 3 | Cell control | 10000 | 10000 | 10000 |
| 4 | Ia | 10800 (90%) | 9600 (80%) | 7200 (60%) |
| 5 | lib | 12000 (100%) | 10800 (90%) | 8400 (70%) |
| 6 | III | 12000 (100%) | 9600 (80%) | 8400 (70%) |
| 7 | IV | 9600 (80%) | 8400 (70%) | 6000(50%) |
| 8 | V | 10800 (90%) | 9600 (80%) | 7200 (60%) |
| 9 | VI | 12000 (100%) | 10800 (90%) | 8400 (70%) |
| 10 | VII | 8400 (70%) | 6000(50%) | 4000 (30%) |
| 11 | VII | 9600 (80%) | 8400 (70%) | 6000(50%) |
| 12 | IX | 9600 (80%) | 8400 (70%) | 6000(50%) |
| 13 | X | 10800 (90%) | 9600 (80%) | 7200 (60%) |
| 14 | XI | 9600 (80%) | 8400 (70%) | 6000 (50%) |
| 15 | XII | 6000 (50%) | 3600 (30%) | 1200 (10%) |
| 16 | XIV | 9600 (80%) | 8400 (70%) | 6000 (50%) |

| | | | | |
|---|---|---|---|---|
| * Concentration of claimed compounds (mg/1) | | | | |
| ** Compound of given concentration was not tested | | | | |
| *** number of cells in 100 fields under consideration | | | | |
| **** the percentage of protection of cells from virus | | | | |

The obtained results presented in Table 3 indicates that all the claimed compounds possess activity against *Herpes virus.*

### Example 12. Determination of effect of the claimed compounds on Chlamydia trachomatis.

The antimicrobial activity of the claimed compounds was studied relative to *C.trachomatis* D323, which is a standard strain from the collection of the chair of microbiology of St. Petersburg State Pavlov Medical University. This strain was derived from a patient with Chlamydia urethritis and has a morphology and physiological activity that are characteristic for representatives of this type; it is sensitive to action of compounds used for treatment of chlamydia infection. Cell cultures McCoy and L929 obtained from the Institute of Cytology of RAS were used in this work [25, 26 -Judson B.A. et al J. of Clinical Microbiology, 1988, vol.26, N12, p.2657-2658; Fenelon L.E. et al. J. of Antimicrobial Chemotherapy, 1990, 26, p.763-767].Concentration of Chlamydia' infectious dose was 1x106 cells/ml. The evaluation of the results was carried out by the way of determination of chlamydial cytoplasmatic inclusions (CPI) that was conducted by the method of immunofluorescence (MicroTrack Chlamydia trachomatis Direct Specimen Test), and by the way of determination of chlamydia antigens that was conducted by means of CylaMonoScreen (Russian-British Joint Venture 66 Regent's Parc Road London NW1 7SX) [26 - Fenelon L.E. et al. J. of Antimicrobial Chemotherapy, 1990, 26, p.763-767].

The effect of the action of preparation was determined by analysis of the monolayer state and number of cells with CPI in comparison to the control (cell culture infected with C.trachomatis D323); in doing so, the number of unchanged cells, which were counted in 100 visual fields obtained using the special net of the microscope eyepiece, was taken into consideration.

**Table 4. Effect of claimed substances on C. trachomatis**

| NN | Compound | Percentage of cells' defense from *C. trachomatis, %* | |
|---|---|---|---|
| | | 100* | 30* |
| 1. | Ib | 80 | 60 |
| 2. | lia | 90 | 70 |
| 3. | III | 100 | 70 |
| 4. | IV | 80 | 50 |
| 5 | V | 80 | 50 |
| 6 | VI | 80 | 50 |
| 7 | VII | 90 | 50 |
| 8 | VIII | 90 | 70 |
| 9 | IX | 80 | 50 |
| 10 | X | 100 | 60 |
| 11 | XI | 90 | 50 |
| 12 | XII | 60 | 40 |
| 13. | XIII | 80 | 60 |

| | | | |
|---|---|---|---|
| * Concentration of claimed compounds (mg/1) | | | |

The data obtained give an evidence of the fact that the claimed compounds can be applied to treat diseases caused by chlamydia.

### Example 13. Anti-influenza virus activity.

Determination of compound's antiviral activity against influenza virus was held on model of chorion-allantois membrane. Compounds in given concentrations was diluted in media for chorion-allantois membrane, injected in wells with fragments of chorion-allantois membrane. After it virus was added and plates were incubated in 33-34°C for 48 hour (for type A virus) and 72 hours (for type 3 virus). Inhibition of viral activity of tested compounds was estimated by haemagglutinating reaction (HA) adding 1% of chicken erythrocytes in culture medium.
Compounds' efficiency was estimated by decrease of viral activity compared to control - index of neutralization (IN). The compound was thought to be ineffective when IN was less than 1,0; and effective if IN was from 1,0 to 2,0 or higher.
Viruses' titer was counted by Rid's and Mench's titers.

**Table 5. Activity of claimed compounds against influenza virus.**

| Compound | Index of neutralization | |
|---|---|---|
| | Influenza virus A | Influenza virus B |
| IB | 0.5 | 0,5 |
| lia | 1,5 | 1,5 |
| III | 0.5 | 1,0 |
| IV | 2,0 | 1,5 |
| V | 1,0 | 1,5 |
| VI | 20 | 1,5 |
| VII | 2,0 | 1,5 |
| VIII | 2,0 | 1,5 |
| IX | 2,0 | 1,5 |
| X | 1,5 | 1,0 |
| XI | 1,5 | 1,5 |

Thus claimed compounds are active against influenza viruses Type A and B.

### Example 14. Activity against HIVs viruses

Activity against HIV virus was determined by protection of T-lymphoblastoid cells MT4 from infecting by virus-containing liquid of HTHIV27 culture. Cells that were infected by viruses were analyzed by the following methods: 1) indirect immunofluorescence (IFA) with polyclonal anti-serum received from HIV infected human (antibody titer in IFA is 1:1000000). Solution 1:540 was used in tests. 2) In competitive IFA with monoclonal antibodies (MonAb) to p24 HIV and polyclonal substrate. Azidothymidin (AZT) (Sigma) was used as control.

**Table 6. Anti-HIV activity of claimed compounds.**

| Compound | Concentration | Level of inhibition of virus reproduction (in %) |
|---|---|---|
| Ib | 100 mg/l | 100 |
| | 50 mg/l | 90 |
| | 5 mg/l | 70 |
| III | 100 mg/l | 100 |
| | 50 mg/l | 90 |
| | 5 mg/l | 70 |
| V | 100 mg/l | 100 |
| | 50 mg/l | 100 |
| | 5 mg/l | 90 |
| XIV | 100 mg/l | 100 |
| | 50 mg/l | 100 |
| | 5 mg/l | 100 |
| XV | 100 mg/l | 100 |
| | 50 mg/l | 100 |
| | 5 mg/l | 90 |
| 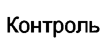 (AZT) | 100 mg/l | 100 |
| | 50 mg/l | 100 |
| | 5 mg/l | 100 |

Thus, tested compounds inhibit reproduction of Virus type 1.

Example 15. The use of claimed compounds combined with anti-HIV compounds Combined effect was estimated by cells' defense adding claimed compounds and azidothymidin. Activity was determined by protection of T-lymphoblastoid cells MT4 from infecting by virus-containing liquid of HTHIV27 culture. Cells that were infected by viruses were analyzed by the following methods: 1) indirect immunofluorescence (IFA) with polyclonal anti-serum received from HIV infected human (antibody titer in IFA is 1:1000000). Solution 1:540 was used in tests. 2) In competitive IFA with monoclonal antibodies (MonAb) to p24 HIV and polyclonal substrate.

**Table 7. Inhibition of immunodeficiency virus' reproduction**

| Compound | Concentration, mg/l | Level of inhibition of virus reproduction (in %) |
|---|---|---|
| Ib | 5.0 | 70 |
| | 0,5 | 20 |
| AZT | 5.0 | 100 |
| | 0,05 | 50 |
| Ib + AZT | 0,5 + 0,05 | 100 |
| XIV | 5 | 100 |
| | 0.5 | 60 |
| Ib + AZT | 0,5 + 0,05 | 100 |

Data received indicate that claimed compounds can be used combined with standard preparation - azidothymidin, used for AIDS' treatment.

### Example 16. Determination of an acute toxicity of the claimed compounds.

The claimed compounds were per orally (1000 mg/kg) or intraperitoneally (200 mg/kg) administrated to no-breed white mice with mass 20-25 g.
Each group consisted of five male and five female mice. The period of observation was 14 days. During the performed investigations there were not observed weight losses, changes in behavior or external appearance character to toxic effect and also cases of animal death. This makes possible to conclude that used concentration of claimed compounds do not possess acute toxicity at used model.

### Industrial Applicability

Examples of synthesis and investigation of physical-chemical characteristics, and biological properties of synthesized compounds confirm the possibility of laboratory and industrial synthesis of all nine claimed compounds by the means that have been leant by modem pharmaceutical industry, and also their clear identification by generally used control methods. Accessible components and usual equipment are used for synthesis of compound that exhibits antiviral and antibacterial activities on the basis of derivatives i.e. 2,8-dithioxo-1H-pyrano[2,3-d; 6,5-d']dipyrimidine and their 10-aza-analogue. This data make conditional upon the satisfying correspondence of the invention to the criteria of "industrially applicability" (IA).

## Claims

1. The inventive substance which exibits antiviral and antibacterial activity is based on derivatives of 2,8-dithioxo-1H-pyrano[2,3-d; 6,5-d']dipyrimidine and 10-aza-analogue, differs in that comprises the derivative of said group of general formula A1 * M: 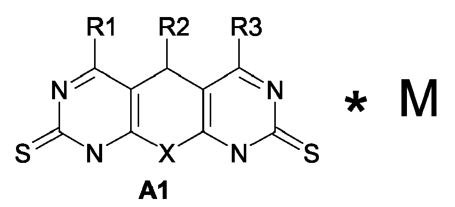 where: X is selected from a group of O, NH, N-Alkyl;
R1 is selected from the group of H, OH, Cl, O-Alkyl, NH₂, NH-Alkyl, NH-Ar, N(Alkyl)₂, SH, S-Alkyl, S-Ar, S-Hetaryl;
R2 is selected from the group of C6H5, Aryl;
R3 is selected from the group of H, Cl, O-Alkyl, NH2, NH-Alkyl, NH-Ar, S-Hetaryl;
M-is absent or is selected from a group: cation Na, K, Li, ammonium or any other pharmacologically acceptable cation or complex of pharmacologically acceptable cation (see above) with anion of one of derivatives of A1 (variants R1-R3 are given above).

2. Compound according to c.1, differs in that X=O, R1=OH, R2=C6H5, R3=Cl, M is absent (Ia).

3. Compound according to c.1, differs in that X=O, R1=OH, R2= C6H4-4-NO2, R3=Cl, M is absent (Ib).

4. Compound according to c. 1, differs in that X=O, R1=R3= Cl, R2= C6H4-4-NO2, M is absent (IIa).

5. Compound according to c. 1, differs in that X=O, R1=OH, R2= C6H4-4-NO2, R3= NH2, M is absent (III).

6. Compound according to c. 1, differs in that X=O, R1=C1, R2= C6H4-4-NO2, R3= NH2, M is absent (IV).

7. Compound according to c. 1, differs in that X=O, R1=R3= NH2, R2= C6H4-4-NO2, M is absent (V).

8. Compound according to c. 1, differs in that X=NH, R1=R3= NH2, R2= C6H4-4-NO2, M is absent (VI).

9. Compound according to c. 1, differs in that X=NH, R1=OH, R2= C6H4-4-C1, R3= NH2, M is absent (VII).

10. Compound according to c. 1, differs in that X=O, R1=OH, R2= C6H4-4-NO2, R3= NHCH3, M is absent (VIII).

11. Compound according to c. 1, differs in that X=O, R1=OH, R2= C6H4-4-NO2, M is absent (IX).

12. Compound according to c. 1, differs in that 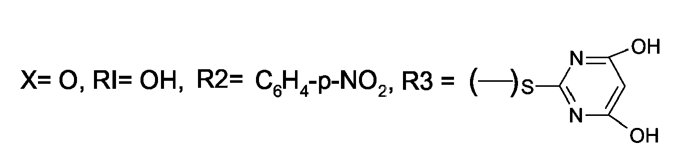 M is absent (X);

13. Compound according to c. 1, differs in that X=O, R1=OH, R2= C6H4-4-NO2, R3= H, M is absent (XI).

14. Compound according to c. 1, differs in that X=O, R1=R3= OH, R2= C6H4-4-NO2, M is absent (XII).

15. Compound according to c. 1, differs in that X=O, R1=OH, R2= C6H4-4-NO2, R3=Cl, M= NH3+A1 according to c. 1, where X=O, R1=R3= OH, R2= C6H4-4-NO2 (XIII).

16. Compound according to c. 1, differs in that X=O, R1=OH, R2= C6H4-4-NO2, R3= NH2, M= NH3+A1 according to c. 1, where X=O, R1=R3= OH, R2= C6H4-4-NO2 (XIV).

17. Compound according to c.1, differs in that X=O, R1=OH, R2= C6H4-4-NO2, R3= H, M= NH3+A1 according to c. 1, where X=O, R1=R3= OH, R2= C6H4-4-NO2 (XV).
